**Europäisches Patentamt**

**European Patent Office**

(19)

**Office européen des brevets**

(11) Publication number : **0 437 079 B1**

(12) # EUROPEAN PATENT SPECIFICATION

(45) Date of publication of patent specification :
27.04.94 Bulletin 94/17

(51) Int. Cl.⁵ : **A61L 27/00, C22C 14/00**

(21) Application number : **90314051.5**

(22) Date of filing : **20.12.90**

(54) **Biocompatible low modulus titanium alloy for medical implants.**

(30) Priority : **21.12.89 US 454181**

(43) Date of publication of application :
**17.07.91 Bulletin 91/29**

(45) Publication of the grant of the patent :
**27.04.94 Bulletin 94/17**

(84) Designated Contracting States :
**AT BE CH DE DK ES FR GB IT LI LU NL SE**

(56) References cited :
**DE-A- 2 703 529**
**US-A- 4 857 269**

(73) Proprietor : **SMITH & NEPHEW RICHARDS INC.**
**1450 Brooks Road**
**Memphis, Tennessee 38116 (US)**

(72) Inventor : **Davidson, James A.**
**2573 Windy Oaks Drive**
**Germantown, TN 38138 (US)**
Inventor : **Kovacs, Paul**
**3227 S. Mendenhall Road**
**Memphis, TN 38115 (US)**

(74) Representative : **Gilholm, Stephen Philip**
**Corporate Patents Department Smith & Nephew Group Research Centre York Science Park**
**Heslington York YO1 5DF (GB)**

EP 0 437 079 B1

Jouve, 18, rue Saint-Denis, 75001 PARIS

## Description

This invention relates to high strength, biocompatible alloys suitable for use as a material for medical prosthetic implants and, in particular a titanium alloy which has a relatively low modulus of elasticity eg. closer to that of bone than other typically-used metal alloys and which alloy does not include any elements which have been shown or suggested as having short term or long term potential adverse effect.

The most common materials used for load-bearing medical implants such as hip or bone prostheses are metallic alloys, ceramics and composites formed of biocompatible polymers and various reinforcing materials.

Polymers are typically used in implants such as intraocular lenses, facial bone remodelling and other non-load bearing applications. In order to use plastics materials in load bearing applications, they are typically reinforced with a particulate or high modulus fibrous material, such as carbon fibre, to produce composites of acceptable strength capable of withstanding relatively great applied loads. Although composites are presently under consideration by many companies, their usefulness as an implant material lies in their relatively low elastic modulus compared to metal and ceramic implants, and their optimum design characteristics are still being explored.

Ceramic prostheses provide excellent biocompatibility, corrosion resistance (inertness) and high compression strength for load-bearing applications. However, ceramic prostheses are typically rigid (high elastic modulus), and unyielding under stress from loads applied during normal use, so that it cannot effectively transfer stresses to surrounding bone. Thus, bone decalcification which results in localised thinning (resorption) and weakening of the bone structure may occur.

Metals and metal alloys such as stainless steel, "Vitalium" (cobalt alloy) and titanium have been used successfully. These materials have the requisite strength characteristics but typically have not been resilient or flexible enough to form an optimum implant material. Also, many alloys contain elements such as aluminum, vanadium, cobalt, nickel, molybdenum, and chromium which studies have suggested might have some long term adverse local or systemic effects on human patients.

Many of the metal alloys typically used in prosthetic implants were developed for other applications such as Ti-6A1-4V in alloy in the aircraft industry. These alloys were later thought to be suitable for use as implant materials because they possess mechanical strength and appeared to have acceptable levels of biocompatibility. However, these metals typically have elastic moduli much higher than that of bone, for example, 316 stainless steel has an elastic modulus of about 200 GPa whilst that of cast heat-treated Co-Cr-Mo alloy is about 240 GPa. Of these, the alloy with the lowest elastic modulus is Ti-6A1-4V with an elastic modulus of about 120 GPa.

It has also been found that many of these metals will corrode to some extent in body fluids thereby releasing ions that might possibly be harmful over a prolonged period of time. It is now believed that the corrosive effects of body fluids is due both to chemical and electro-chemical processes, with corrosion products forming when certain commonly used metal alloys ionise from corrosion processes in the body. For example, aluminum metal ions have been associated with Alzheimer's disease and vanadium, cobalt, molybdenum, nickel and chromium are suspected of being toxic or carcinogenic.

It has been suggested that metals could be coated with a biocompatible plastic, ceramic or oxide to overcome the corrosion problem. However, coatings may abraid and tend to wear off, especially on articulating bearing surfaces of total joints, and are susceptible to galling and separating from the metal substrate, exposing the metal to body fluids.

Generally, it is the industry practice to passivate the implant metal alloys. However, passivation produces only thin amorphous, poorly attached protective oxide films which have not proved totally effective in eliminating the formation of corrosion products in the body, ie. relative motion against adjacent bone particularly in situations where fretting occurs in the body.

Titanium alloys offer advantages over the stainless steels because of the superior biocompatbility low susceptibility to corrosion in the body coupled with their high strength and relatively low modulus of elasticity. Upon cooling, the currently used Ti-6A1-4V alloy transforms from a β-structure to an α- plus β-structure at about 1000°C. This transition can be shifted to a lower temperature by the addition of one or more suitable β-phase stabilisers such as molybdenum, zirconium, niobium, vanadium, tantalum, cobalt, chromium, iron, manganese and nickel.

Some efforts have been directed toward the development of alloys that eliminate harmful metals. For example, US Patent 4040129 to Steinemann et al, is directed to an alloy which includes titanium or zirconium as one component and, as a second component, any one or more of: nickel, tantalum, chromium, molybdenum or aluminum, but does not recognise or suggest any advantages from having a relatively low elastic modulus, or advantages or disadvantages associated with property changes which can occur during high temperature sintering treatments (at about 1250°C), commonly employed to attach porous metal coatings into which bone can grow to stabilise non-cemented, press-fit devices into the skeletal structure. Steinemann also indicates that the ultimate tensile strength should be greater than about 979 MPa (142 ksi) with a minimum tensile elongation of 10%.

Although the disclosure in US Patent No. 4040129 provides that copper, cobalt, nickel, vanadium and tin should be excluded, apart from their presence as unavoidable impurities, it is indicated that it is permissible to have chromium, molybdenum and/or aluminum, which are all believed to have potential long-term adverse effects, present in the alloy as long as their combined weight does not exceed 20% of the total weight of the alloy.

US Patent 4857269 to Wang et al. relates to a titanium alloy for a prosthetic implant said to have high strength and a low modulus. The titanium alloy contains upto 24 wt.% of at least one isomorphous beta stabiliser from the group molybdenum, tantalum, zirconium, and niobium; upto 3 wt.% of at least one eutectoid beta stabiliser from the group iron, manganese, chromium, cobalt or nickel; and optionally upto 3 wt.% of a metallic $\alpha$-stabiliser from the group aluminium and lanthanum. Incidental impurities upto 0.05% carbon, 0.30% oxygen, 0.02% nitrogen and upto 0.02% of the eutectoid former hydrogen are also included. Although there is some discussion of having an elastic modulus (eg. Young's modulus) around 85 GPa, the only examples of a low modulus (66.9-77.9 GPa) all contain 11.5 wt.% Mo which is a potentially toxic element and undesirable for optimising biocompatibility. Thus although the elastic modulus is discussed, aspects of optimum biocompatibility are not addressed.

Other currently used metal alloys have similar drawbacks. For example, the commonly used Ti-6A1-4V alloy, with appropriate heat treatment, offers some degree of biocompatibility but has an elastic modulus of about 120 GPa. Although this elastic modulus is lower than other currently used implant alloys and accordingly offers better load transfer to the surrounding bone, this modulus is still significantly greater than desired. Moreover, the alloy contains aluminum and also vanadium, which are suspected to be a toxic or carcinogenic material when present in sufficient quantity.

A commercially available Ti-6A1-7Nb alloy is PROTOSUL 100 (Sulzer Bros. Ltd.) which intentionally avoids the potentially adverse effects of vanadium toxicity by substituting niobium. However, the alloy still contains aluminum and has an elastic modulus of about 110 GPa ($15.9 \times 10^6$ psi) in the heat-treated condition, and has a tensile strength of about 1060 MPa.

With orthopaedic prostheses being implanted in younger people and remaining in the human body for longer periods of time, there is a need for an implant material with requisite strength and flexibility requirements, which does not contain elements which are suspected of having long-term harmful effects on the human body.

The present invention proposes alloys useful in the manufacture of orthopaedic implants, which possess the characteristics of relatively high strength, exceptionally low modulus of elasticity, and are free from any potentially toxic elements, and an implant formed from such alloys.

In accordance with the present invention there is provided a low modulus biocompatible prosthetic implant comprising an alloy having the following alloying components:
a) titanium;
b) 10 to 20 wt.% or 35 to 50 wt.% niobium and;
c) upto 20 wt.% zirconium and/or tantalum,
wherein other elements are excluded except for those trace amounts of impurities and interstitials present in the alloying components or picked up during fabrication.

The present invention further provides a process for producing a prosthetic implant, comprising:
a) selecting an alloy comprising of titanium; from 10 to 20 wt.% or from 35 to 50 wt.% niobium; and upto 20 wt.% zirconium and/or tantalum;
b) shaping an implant from the alloy; and
c) age hardening the implant.

In another embodiment of the invention there is provided a low modulus biocompatible alloy useful for prosthetic implants consisting essentially of the following alloying components titanium, niobium, zirconium and/or tantalum wherein the niobium is present in an amount of from 10 to 20 wt.% or from 35 to 50 wt.% and the zirconium and/or tantalum is present in amount of upto 20 wt.% and wherein other elements are excluded except for those trace amounts of impurities and interstitials present in the alloying components or picked up during fabrication.

The alloys of the present invention are titanium-niobium alloys, optionally containing upto 20% by weight of zirconium and/or tantalum. Other elements are not deliberately added, but may be present in trace amounts to the extent that they were present as unavoidable impurities in the metals used to produce the alloy. Other non-toxic filler materials such as tantalum, which could be used to stabilise the β-phase, but not affect the low modulus, eg. maintain it less than 85 GPa, could also be used. The exclusion of elements beside titanium, zirconium and niobium or tantalum results in an alloy which does not contain known toxins or carcinogens or ele-

3

ments that are known or suspected of inducing adverse tissue or other systemic or local biological response in the long term.

In producing the implants of the invention titanium alloy is rapidly cooled from above the β-transus and aged to provide adequate strength. This is normally performed after the implant has been shaped from the non-hardened alloy. Further, such alloys have a low modulus of elasticity, even after high-temperature sintering to attach porous-coatings, for example of 62-75 GPa. This compares favourably with the elastic modulus of fibre reinforced polymer composite implants, which are typically in the range 60-70 GPa and can be as high as 85 GPa for strength adequate for long-term in-vivo loading, and is a significant improvement over Ti-6A1-4V which has a modulus of elasticity of 120 GPa.

In certain applications it may still be desirable to coat the alloy surface eg. when formed into an implant with wear-resistant coatings such as amorphous diamond-like carbon coatings, zirconium dioxide coatings, titanium nitrides, carbides, or the like for protection against potential micro-fretting, such as might occur on the bearing surfaces of implant prostheses.

A porous coating, such as a bead or wire mesh coating amy also be attached to implants. Such coatings are often useful to provide interstitial spaces for tissue ingrowth into the implant, which tends to stabilise the implant in the skeletal structure. Further, even though the application of such porous coatings usually requires sintering at relatively high temperatures, the properties of the alloy that might affect its usefulness as an implant material are not adversely affected.

While prostheses fabricated from the invention alloy posses a relatively high strength, the usefulness of these prostheses is not limited to load-bearing applications. Because of its corrosion resistance and non-toxicity and relatively low modulus of elasticity, the alloy can be used to fabricate many types of orthopaedic implants including, but not limited to, hip joints, knee joints, cheek bones, tooth implants, skull plates, fracture plates, intramedullary rods, staples, bone screws and other implants which may not necessarily require optimum strength.

The alloys used for the manufacture of the implants of the invention may be produced by combining, as commercially pure components, titanium, and niobium and optionally zirconium and/or tantalum in the appropriate proportions. The methods for titanium alloy production, such as casting, powder metallurgy, et., are well known to those of ordinary skill in the art of metallurgy and the production of the alloy requires no special skills or precautions beyond the materials, proportions and techniques described below.

The alloys of the invention contains titanium as the major component and may comprise 85 wt.% of the alloy in combination with 13 wt.% of niobium. Preferred zirconium containing alloys may comprise at least 74% Ti (eg. 74% Ti, 13% Nb and 13% Zr). While tantalum may be substituted for niobium to stabilise the β-phase titanium, niobium is the preferred component due to its effect of lowering the elastic modulus of the alloy when present in certain specific proportions. Other elements are not deliberately added to the alloy but may be present in such quantities that occur as impurities in the commercially pure titanium, zirconium, niobium or tantalum used to prepare the alloy and such contaminants as may arise from the melting (alloying) process. Non-toxic filler materials, such as tantalum, could also be added to reduce the β-transus (stabilise β) and improve strength as long as the relatively low modulus of elasticity (less than 85 GPa) of the base alloy is not significantly affected.

While the as-cast or powder metallurgically prepared alloy can be used as an implant material, it can optionally be mechanically hot rolled at 825-875°C. After cooling, it can then be reheated to 875°C for 20 minutes and then quenched with water. This reheating step may be eliminated if the alloy is quenched rapidly from the hot working temperature. These hot rolling, cooling, reheating and quenching steps develop the cast alloy into a wrought material having a finer grain than the as-cast or powder metallurgically prepared alloy and renders it more suitable for use as an implant material.

The inventive alloy, in this hot rolled, reheated and quenched form, has an elastic modulus of 60 to 65 GPa, a tensile strength of 700 GPa and an elongation of 25%. While such an alloy might be suitable for use in a variety of implant applications, it is desirable that alloys used in more severe load-bearing implant applications have a greater strength as well as a lower elastic modulus (less than 85 GPa).

As used herein the term "high strength" refers to an alloy having a tensile strength above at least 620 MPa. Apt alloys have a tensile strength of upto 1050 MPa, preferably from 890 to 1050 MPa, more preferably from 895 to 1000 MPa.

The term "low modulus" as used herein refers to a Young's modulus below 85 GPa. Aptly the modulus will be at least 60 GPa, suitably from 65 to 76 GPa, preferably from 70 to 76 GPa.

Although the hot rolled, reheated and quenched alloy is a suitable implant material, its properties can be improved by forging or cold working, or by an ageing heat treatment or a combination of these. Ageing treatment can increase the strength and hardness of the material, and reduce its elongation while maintaining a relatively low modulus of elasticity. The treatment can be varied to obtain the desired properties.

In titanium alloys, the niobium (or tantalum, if this element is added) acts to stabilise the β-phase since it is a β-isomorphous phase stabiliser. This results in a lower β-phase transus temperature and upon rapid cooling from about the β-transus temperature, the presence of a greater proportion of the β-phase titanium in the alloy microstructure. This enhances the ability of the alloy to harden on subsequent ageing.

Niobium, in particular, when present in preferred quantities of from 6 to 10 atomic percent (most preferably 8 atomic percent) or in an alternative preferred range of from 22 to 32 atomic percent, produces a low modulus composition when alloyed with titanium. Deviation from these ranges of niobium concentration tends to increase the elastic modulus. In weight percent terms, these preferred compositional ranges of niobium in the titanium-zirconium alloy translate to 10 to 20 wt.% and 35 to 50 wt.%.

Titanium alloys containing 13 wt.% niobium correspond to those having about 8 atomic percent niobium. Thus, the Ti-13Nb-13Zr alloy is believed to identify an apt low modulus, titanium alloy composition.

As previously mentioned, tantalum may be substituted for niobium to stabilise the β-phase, but niobium is preferred due to its effect in reducing the elastic modulus. Substitution with zirconium can improve strength.

Whereas the niobium proportion is critical to obtain the desired low modulus property, the zirconium proportion is not as critical. It is desirable to maintain the proportion of zirconium at less than 20 wt.% but higher proportions also may be useful.

Zirconium, it is believed, is capable of stabilising both α- and β-phase titanium alloy, but acts by being in solution in the alloy as a β-stabiliser by slowing the transformation process in the inventive alloy. It is further believed that the larger ionic radius of zirconium (35% larger than that of titanium) helps to disrupt ionic bonding forces in the alloy resulting in some reduction in the modulus of elasticity.

In order to effect the transition to the β-phase, the alloy may be treated by heating to 875°C for 20 minutes. Lower temperatures above the β-transus may also be used. The β-phase may also be induced by heating to higher temperatures for shorter periods of time. The critical factor is heating to at least above the β-transition temperature, about 728°C for Ti-13Zr-13Nb, for a period of time sufficient to obtain a substantial conversion of the titanium alloy to the β-phase prior to cooling to room temperature. Conversion of the alloy the β-phase and cooling may be effected before, during or after shaping for implantation and sintering of a porous metal coating, whichever is most convenient.

Based upon the foregoing, it is apparent that the titanium proportion of certain embodiments of the invention alloy could be less than 50 wt.%. Nevertheless, these alloys are, for purposes of the specification and claims, referred to as "titanium alloys." For example, a titanium alloy may contain 20 wt.% zirconium and 45 wt.% niobium with only 35 wt.% titanium.

The machining, casting or forging of the alloy into the desired implant shape may be carried out by any of conventional methods used for titanium alloys. Further, implants could be pressed from the powdered alloy under conditions of heat and pressure in pre-forms in the shape of the desired implant. Conventional sintering and hot isostatic pressure treatments can be readily applied.

While the alloy provides a non-toxic prosthesis, it may yet be desired for other reasons such as micro-fretting against bone or polyethylene bearing surfaces to coat the prosthesis. In this event, the surface of the prosthesis may be coated with an amorphous diamond-like carbon coating or ceramic-like coating such as titanium nitride or titanium carbide using chemical or plasma vapour deposition techniques to provide a hard, impervious, smooth surface coating. These coatings are especially useful if the prosthesis is subjected to conditions of wear, such as, for instance, in the case of bearing surfaces of knee or hip prostheses. For purposes of abrasion resistance only, routine commercially available surface diffusion hardening can performed such as carbonisation, nitriding or oxygen ion diffusion.

Methods for providing hard, low-friction, impervious biocompatible amorphous diamond-like carbon coatings are known in the art and are disclosed in, for example, EP 302717-A to Ion Tech, and Chemical Abstracts 43655P, Vol. 101 describing Japan Kokai 59/851 to Sumitomo Electric.

Implants of the invention may be supplied with a porous bead or wire coating of titanium alloy of the same or different composition including pure titanium to allow stabilisation of the implant in the skeletal structure of the patient after implantation by bone ingrowth into the porous structure. Such porous structures are normally attached to the implant surface by sintering. This involves heating the implant to above 1250°C. The mechanical properties of titanium alloys can change significantly due to substantial grain growth and other metallurgical factors arising from the sintering process. Diffusion bonding under pressure can be performed at high temperatures, for example from 900 to 1000°C. Thus, after sintering to attach the porous coating, it is preferred that the Ti-13Zr-13Nb implant be reheated to 875°C (or above the β-transus) for 20-40 minutes then quenched before being aged at 500°C for 6 hours to restore mechanical properties. If quenched adequately from the sintering temperature, it may be possible to go directly to the ageing process.

The present invention will be illustrated by reference to the following examples and to the accompanying drawings in which:

Figure 1. is a schematic drawing of hip joint stem with a porous coating.

Figure 2. is a bar graph comparing the mechanical properties of an invention alloy with other materials and bone.

Figure 3. is a bar graph comparing the mechanical properties of an invention alloy with other materials and bone.

Figure 4. shows the effect of various techniques of age hardening on two different invention alloys.

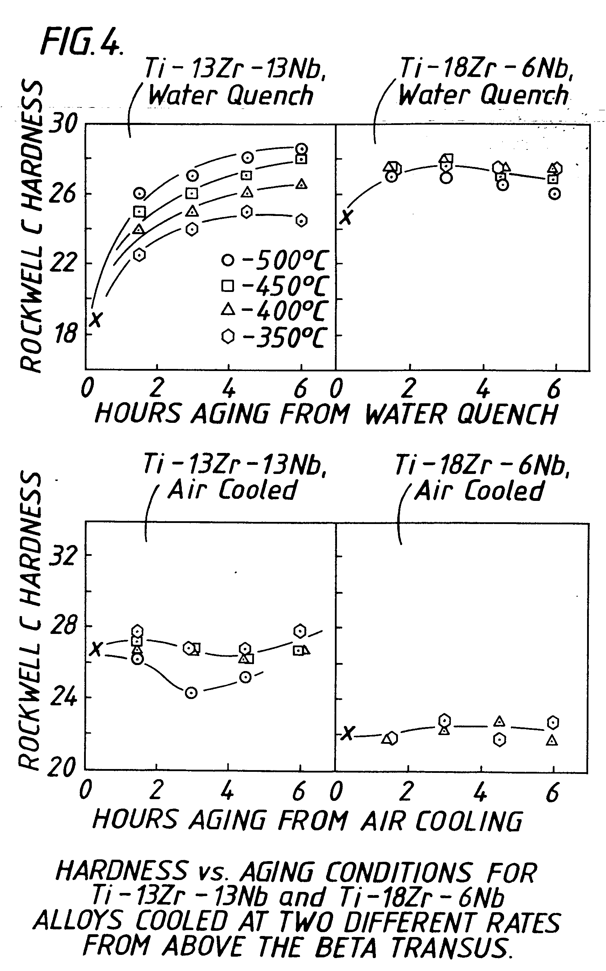

The ageing temperature used in the examples is determined to be acceptable, but not necessarily optimal, based on the hardness versus ageing response shown in Figure 4.

Example 1

An alloy including, by weight, 74% titanium, 13% niobium and 13% zirconium, was hot rolled at a temperature in the range 825-875°C to 14 mm thick plate. The plate was cooled to room temperature then reheated to 875°C where it was maintained for 20 minutes and then water quenched to room temperature. The β-transus for this alloy was determined to be about 728°C as compared to about 1000°C for Ti-6A1-V. The mechanical properties of the heat-treated, quenched Ti-Zr-Nb alloy, which has an acicular transformed β-structure, are shown in Table I.

TABLE I

Mechanical Properties of Ti-13Zr-13Nb

As water Quenched from Hot Rolling Temperature

| | |
|---|---|
| Tensile Strength | 710 MPa |
| Yield Strength | 476 MPa |
| Elongation | 26% |
| Reduction in area | 70% |
| Young's Modulus | 62 GPa |
| Rockwell C Hardness | 18-19 |

Example 2

The heat-treated, quenched Ti-Zr-Nb alloy of Example 1 was aged by heating at 500°C for 6 hours. The mechanical properties of this aged alloy as shown in Table II.

## TABLE II

### Mechanical Properties of Quenched

### Ti-13Zr-13Nb Aged 500°C for Six Hours

| | |
|---|---|
| Tensile Strength | 917 MPa |
| Yield Strength | 796 MPa |
| Elongation | 13% |
| Reduction in area | 42% |
| Young's Modulus | 76.6 GPa |

Example 3

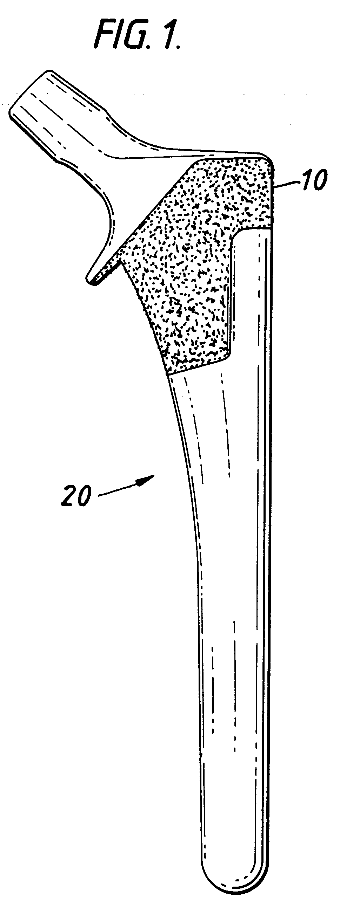

Samples of the alloy of Example 1 were sintered at about 1250°C to attach a porous titanium bead coating of the type shown in Figure 1. The bead-coated alloy samples of were then reheated to 875°C and maintained at this temperature for 40 minutes before being water-quenched. A group of six samples were aged at 500°C for 6 hours and the mechanical properties of aged and non-aged samples (three each) were tested and are shown in Table III.

## TABLE III

### Mechanical Properties of Ti-13Zr-13Nb Alloy

### Following Bead Sintering, Reheating to

### 875°C, and Water Quenched

| | As-Quenched (Avg.) | Aged (500°C Six Hours) |
|---|---|---|
| Tensile Strength | 664MPa | 900 MPa |
| Yield Strength | 465 MPa | 795 MPa |
| Elongation | 20% | 4% |
| Reduction in area | 46% | 9% |
| Young's Modulus | 61.8 GPa | 74.7 GPa |

Note that the sintering treatment can significantly alter the mechanical properties, particularly ductility. Thus, an alloy acceptable for a particular application in unsintered form may not necessarily be effective in that application following a high-temperature sintering treatment routinely used to attach a porous titanium coating. To minimise these effects, lower temperature diffusion bonding methods can be used in which a sintering temperature near the β-transus may be effective. Alternatively, pre-sintered porous metal pads can be tack-welded to the implant.

7

Example 4

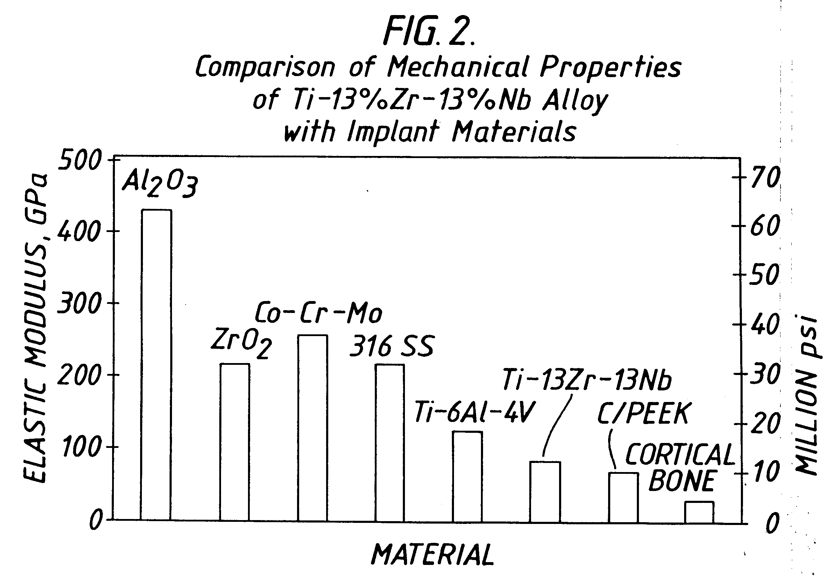

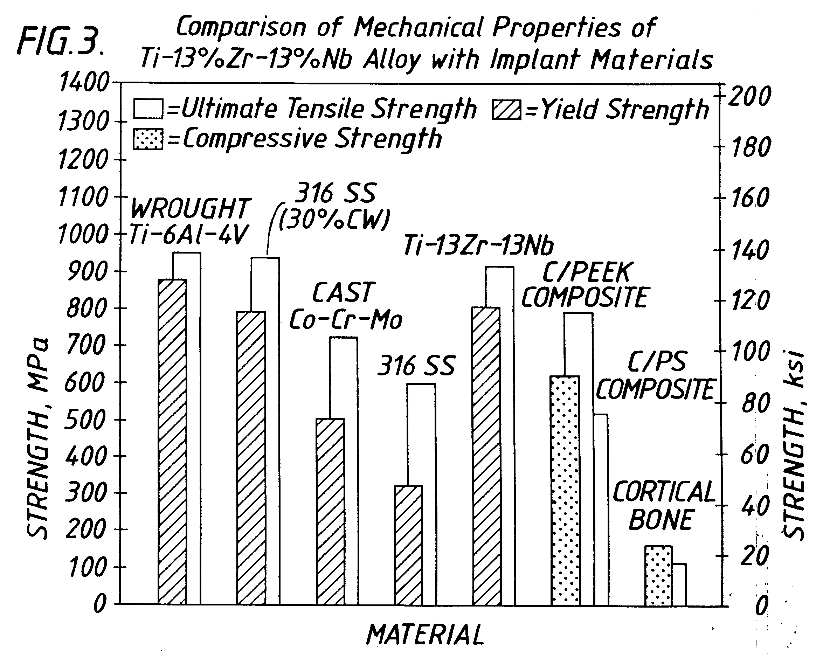

A comparison of the elastic modulus, tensile strength and yield strength of the Ti-13Zr-13Nb invention alloy with those of known alloys, composites and cortical bone, are summarised in Figures 2 and 3. $Al_2O_3$ and $ZrO_2$ refer to ceramics while C/PEEK refers to a carbon reinforced polyetheretherketone composite and C/PS refers to a carbon reinforced polysulfone composite. All the mechanical property data of Figures 2 and 3 were obtained from literature sources except for the data pertaining to the invention alloy which were measured using standard ASTM tensile testing techniques. It is significant that the Ti-13Zr-13Nb invention alloy has an elastic modulus similar to carbon fibre reinforced composites and closer to that of bone than the other metals (Figure 2) while at the same time possessing a strength comparable to or better than other metals (Figure 3). Tensile strength can be further increased (for example to about 1050 MPa or greater) by hot or cold working (or forging) prior to quenching and ageing.

Example 5

An alternative low modulus biocompatible comprising Ti-18Zr-67Nb was also evaluated for mechanical properties.

A sample of Ti-18Zr-6Nb was sintered to attach a porous metal coating. Thereafter, the sintered alloy was reheated to 875°C, ie. above the β-transus, and water quenched. The properties of the as-quenched alloy are shown in Table IV. The sample was then aged at 450°C for 3 hours and tested. These results are also shown in Table IV.

As compared to the Ti-13Zr-13Nb alloy of Example 3, this alloy's modulus of elasticity is not as low as the Ti-13Zr-13Nb alloy but is still lower than that of Ti-6A1-4V. Further, the Ti-18Zr-6Nb alloy has a relatively low β-transus, 760°C compared to that of Ti-6A1-4V which is 1000°C.

## TABLE IV

### Mechanical Properties of Ti-18Zr-6Nb Following a High Temperature Sintering Treatment, Reheating to 875°C, and Water Quenching and Aging

|  | As-Quenched | Aged 450°C 3 Hours |
|---|---|---|
| Tensile Strength | 807 MPa | 876 MPa |
| Yield Strength | 659 MPa | 733 MPa |
| Elongation | 8% | 8% |
| Reduction in area | 26% | 28% |
| Young's Modulus | 85.2 GPa | 86.8% |

Note that because of the less than optimum niobium content, the elastic modulus is not as low as the previous example. Thus, proper selection of niobium content is important for optimising the low elastic modulus. However, the presence of zirconium helps to keep the elastic modulus at an acceptably low level (less than 85 GPa).

Example 6

The effect of ageing conditions on Ti-13Zr-13Nb and Ti-18Zr-6Nb was investigated. Separate samples of each alloy were air-cooled or water-quenched from above the β-transus, aged at 500, 450, 400 and 350°C for upto 6 hours then air-cooled. The results are recorded in Figure 4.

Example 7

A femoral prosthesis, as shown in Figure 1, was produced by forging and machining an alloy as described in Example 1. Onto the stem region 10 of the prosthesis 20, was sintered a titanium bead coating and thereafter age-hardened as described in Example 2.

The tensile strength of the implant was found to be 875 MPa and the modulus 75 GPa.

Example 8

An alloy having the composition described in Example 1 was forged and air cooled to produce a hip prosthesis as illustrated in Figure 1. Upon forging the product was water quenched then aged. The modulus of the aged product was determined to be 80 GPa and the tensile strength was 1000 MPa.

## Claims

1. A low modulus biocompatible prosthetic implant comprising an alloy having the following alloying components:
   a) titanium;
   b) 10 to 20 wt.% or 35 to 50 wt.% niobium and;
   c) upto 20 wt.% zirconium and/or tantalum,
   wherein other elements are excluded except for those trace amounts of impurities and interstitials present in the alloying components or picked up during fabrication.

2. An implant according to claim 1 wherein the alloying components are 84 wt.% titanium and 16 wt.% niobium.

3. An implant according to any one of the preceding claims wherein the alloying components are 74 wt.% titanium, 13 wt.% niobium, 13 wt.% zirconium.

4. An implant according to any one of the preceding claims wherein the alloy comprises zirconium and/or tantalum in an amount not exceeding 20% by weight in total.

5. An implant according to any one of the preceding claims wherein the implant has an elastic modulus of from 60 to 90 GPa.

6. An implant according to any one of the preceding claims wherein the prosthesis includes at least a partial inner or outer surface coating or treatment comprising oxides, nitrides or carbides of the elements selected from the group consisting of titanium, niobium and zirconium and/or tantalum.

7. An implant according to any one of the preceding claims wherein the prosthesis includes at least a partial outer surface coating of an amorphous diamond-like carbon material.

8. A process for producing a prosthetic implant, comprising:
   a) selecting an alloy comprising of titanium; from 10 to 20 wt.% or from 35 to 50 wt.% niobium; and upto 20 wt.% zirconium and/or tantalum;
   b) shaping an implant from the alloy; and
   c) age hardening the implant.

9. The process of claim 8 comprising the steps of hot rolling or forging the alloy before shaping the implant and ageing.

10. The process of claim 8 comprising cold working below the β-transus prior to ageing.

11. The process of claims 8 or 9 wherein the steps of age hardening includes heating the alloy to above the β-transus for 20 minutes and rapidly cooling to room temperature.

12. The process of any one of claims 8 to 11 further comprising the step of age hardening the alloy at between 300 and 600°C for a period of time to optimise strength and ductility.

13. A process according to any one of claims 8 to 12 comprising the step of coating at least a portion of the prosthesis with a porous coating of beads or wire mesh to form a porous tissue ingrowth coating.

14. A process according to any one of claims 8 to 13 further comprising forming an outer solid coating over at least a portion of the prosthesis.

15. A low modulus biocompatible alloy useful for prosthetic implants consisting essentially of the alloying components titanium, niobium, zirconium and/or tantalum wherein the niobium is present in an amount of from 10 to 20 wt.% or from 35 to 50 wt.% and the zirconium and/or tantalum is present in amount of upto 20 wt.% and wherein other elements are excluded except for those trace amounts of impurities and interstitials present in the alloying components or picked up during fabrication.

16. The alloy of claim 15 having an elastic modulus of from 60 to 90 GPa.

17. The alloy of claim 15 or claim 16 consisting essentially of 74 wt.% titanium, 13 wt.% niobium, 13 wt.% zirconium.

18. The alloy of claims 15 or 16 consisting essentially of 84 wt.% titanium and 16 wt.% niobium.

19. The alloy of claim 15 or 16 having 1.2% by weight tantalum.

20. An alloy as claimed in any one of claims 15 to 18 having sub-surface hardening by oxide diffusion to a depth of upto 100 μm.


**Patentansprüche**

1. Biokompatibles, prothetisches Implantat mit niedrigem Modul, umfassend eine Legierung mit den folgenden Legierungsbestandteilen:
   a) Titan
   b) 10 bis 20 Gew.-% oder 35 bis 50 Gew.-% Niobium und
   c) bis zu 20 Gew.-% Zirkonium und/oder Tantal,
   in welcher andere Elemente außer den Spurenmengen an Verunreinigungen und Zwischengittermaterialien, welche in den Legierungsbestandteilen vorhanden sind oder während der Herstellung aufgenommen wurden, ausgeschlossen sind.

2. Implantat gemäß Anspruch 1, bei welchem die Legierungsbestandteile 84 Gew.-% Titan und 16 Gew.-% Niobium sind.

3. Implantat gemäß einem der vorhergehenden Ansprüche, bei welchem die Legierungsbestandteile 74 Gew.-% Titan, 13 Gew.-% Niobium und 13 Gew.-% Zirkonium sind.

4. Implantat gemäß einem der vorhergehenden Ansprüche, bei welchem die Legierung Zirkonium und/oder Tantal in einer insgesamt 20 Gew.-% nicht überschreitenden Menge umfaßt.

5. Implantat gemäß einem der vorhergehenden Ansprüche, bei welchem das Implantat ein elastisches Modul von 60 bis 90 GPa besitzt.

6. Implantat gemäß einem der vorhergehenden Ansprüche, bei welchem die Prothese wenigstens einen teilweisen inneren oder äußeren Oberflächenüberzug oder -behandlung einschließt, welche Oxide, Nitride oder Carbide der Elemente umfassen, welche aus der aus Titan, Niobium und Zirkonium und/oder Tantal bestehenden Gruppe ausgewählt sind.

7. Implantat gemäß einem der vorhergehenden Ansprüche, bei welchem die Prothese wenigstens einen teilweisen äußeren Oberflächenüberzug aus einem amorphen, diamantartigen Kohlenstoffmaterial umfaßt.

8. Verfahren zum Herstellen eines prothetischen Implantats, umfassend:

a) Auswählen einer Legierung, welche Titan, 10 bis 20 Gew.-% oder 35 bis 50 Gew.-% Niobium und bis zu 20 Gew.-% Zirkonium und/oder Tantal umfaßt,

b) Formen eines Implantats aus der Legierung und

c) Aushärtenlassen des Implantats.

9. Verfahren des Anspruchs 8, welches die Schritte des Warmwalzens oder Schmiedens der Legierung vor dem Formen des Implantats und dem Aushärtenlassen umfaßt.

10. Verfahren des Anspruchs 8, welches vor dem Altern eine Kaltverformung unter dem β-Umwandlungspunkt umfaßt.

11. Verfahren der Ansprüche 8 oder 9, bei welchem der Schritt des Aushärtenlassens 20 Minuten Erwärmen der Legierung über den β-Umwandlungspunkt und das rasche Kühlen auf Raumtemperatur umfaßt.

12. Verfahren einer der Ansprüche 8 bis 11, welches weiter den Schritt des Aushärtenlassens der Legierung zwischen 300 und 600°C über einen Zeitraum zum Optimieren der Festigkeit und Verformbarkeit umfaßt.

13. Verfahren gemäß einem der Ansprüche 8 bis 12, welches den Schritt des Überziehens wenigstens eines Teils der Prothese mit einer porösen Beschichtung aus Kugeln oder Drahtgeflecht unter Bilden eines porösen Überzugs zum Gewebeeinwuchs umfaßt.

14. Verfahren gemäß einem der Ansprüche 8 bis 13, welches weiter das Bilden eines äußeren, festen Überzugs auf wenigstens einem Teil der Prothese umfaßt.

15. Biokompatible Legierung mit niedrigem Modul, welche für prothetische Implantate brauchbar ist und im wesentlichen aus den Legierungsbestandteilen Titan, Niobium, Zirkonium und/oder Tantal besteht, in welcher das Niobium in einer Menge von 10 bis 20 Gew.-% oder von 35 bis 50 Gew.-% vorhanden ist und das Zirkonium und/oder Tantal in einer Menge von bis zu 20 Gew.-% vorhanden ist und in welcher andere Elemente außer jenen Spurenmengen an Verunreinigungen und Zwischengittermaterialien, welche in den Legierungsbestandteilen vorhanden sind oder während der Herstellung aufgenommen wurden, ausgeschlossen sind.

16. Legierung des Anspruchs 15 mit einem elastischen Modul von 60 bis 90 GPa.

17. Legierung des Anspruchs 15 oder Anspruchs 16, welche im wesentlichen aus 74 Gew.-% Titan, 13 Gew.-% Niobium und 13 Gew.-% Zirkonium besteht.

18. Legierung der Ansprüche 15 oder 16, welche im wesentlichen aus 84 Gew.-% Titan und 16 Gew.-% Niobium besteht.

19. Legierung des Anspruchs 15 oder 16 mit 1,2 Gew.-% Tantal.

20. Legierung wie in einem der Ansprüche 15 bis 18 beansprucht mit einer Härtung durch Oxiddiffusion unter der Oberfläche in einer Tiefe von bis zu 100 μm.

**Revendications**

1. Implant de prothèse biocompatible de bas module, comprenant un alliage ayant les composants d'alliage suivants :

a) titane ;

b) 10 à 20% en poids ou 35 à 50% en poids de niobium; et

c) jusqu'à 20% en poids de zirconium et/ou de tantale ;

dans lequel les autres éléments sont exclus à l'exception des impuretés à l'état de traces et des matières interstitielles qui sont présentes dans les composants de l'alliage ou captées pendant la fabrication.

2. Implant suivant la revendication 1, dans lequel les composants de l'alliage sont 84% en poids de titane et 16% en poids de niobium.

3. Implant suivant une quelconque des revendications précédentes, dans lequel les composants de l'alliage sont 74% en poids de titane, 13% en poids de niobium et 13% en poids de zirconium.

4. Implant suivant une quelconque des revendications précédentes, dans lequel l'alliage comprend du zirconium et/ou du tantale en unequantité ne dépassant pas 20% en poids au total.

5. Implant suivant une quelconque des revendications précédentes, dans lequel l'implant a un module d'élasticité de 60 à 90 GPa.

6. Implant suivant une quelconque des revendications précédentes, dans lequel la prothèse comprend au moins un revêtement ou un traitement partiel de la surface intérieure ou extérieure, comprenant des oxydes, des nitrures ou des carbures des éléments choisis dans le groupe composé du titane, du niobium et du zirconium et/ou tantale.

7. Implant suivant une quelcoqnue des revendications précédentes, dans lequel la prothèse comprend au moins un revêtement partiel de la surface extérieure en une matière de type carbone amorphe analogue au diamant.

8. Procédé de fabrication d'un implant de prothèse, comprenant :
   a) la sélection d'un alliage composé de titane ; de 10 à 20% en poids ou de 35 à 50% en poids de niobium; et jusqu'à 20% en poids de zirconium et/ou de tantale ;
   b) la formation d'un implant à partir de l'alliage ; et
   c) le durcissement par vieillissement de l'implant.

9. Procédé suivant la revendication 8, comprenant les étapes de laminage à chaud ou de forgeage de l'alliage avant la formation et le vieillissement de l'implant.

10. Procédé suivant la revendication 8, comprenant un travail à froid au-dessous de la transition β avant le vieillissement.

11. Procédé suivant la revendication 8 ou 9, dans lequel l'étape de durcissement par vieillissement comprend le chauffage de l'alliage au-dessus de la transition β pendant 20 minutes et le refroidissement rapide à température ambiante.

12. Procédé suivant une quelconque des revendications 8 à 11, comprenant en outre l'étape de durcissement par vieillissement de l'alliage à une température comprise entre 300 et 600°C pendant une durée adéquate pour optimiser la résistance et la ductilité.

13. Procédé suivant une quelconque des revendications 8 à 12, comprenant l'étape de revêtement d'au moins une partie de la prothèse avec un revêtement poreux de perles ou de grillage métallique , pour former un revêtement poreux pour croissance interne d'un tissu.

14. Procédé suivant une quelconque des revendications 8 à 13, comprenant en outre la formation d'un revêtement extérieur solide sur au moins une partie de la prothèse.

15. Alliage biocompatible de bas module utilisable pour des implants de prothèse, qui comprend essentiellement comme composants d'alliage le titane, le niobium, le zirconium et/ou le tantale, dans lequel le niobium est présent en une quantité comprise entre 10 et 20% en poids ou entre 35 et 50% en poids et le zirconium et/ou tantale est présent en une quantité atteignant 20% en poids, et dans lequel les autres éléments sont exclus à l'exception des traces d'impuretés et de matières interstitielles qui sont présentes dans les composants de l'alliage ou qui sont captées pendant la fabrication.

16. Alliage suivant la revendication 15, ayant un module d'élasticité de 60 à 90 GPa.

17. Alliage suivant la revendication 15 ou 16, composé essentiellement de 74% en poids de titane, 13% en poids de niobium et 13% en poids de zirconium.

18. Alliage suivant la revendication 15 ou 16, composé essentiellement de 84% en poids de titane et de 16% en poids de niobium.

19. Alliage suivant la revendication 15 ou 16, ayant 1,2% en poids de tantale.

20. Alliage suivant une quelconque des revendications 15 à 18, ayant un durcissement sub-superficiel par diffusion d'oxyde à une profondeur pouvant atteindre 100μm.

# FIG. 1.

## FIG. 2.
### Comparison of Mechanical Properties of Ti-13%Zr-13%Nb Alloy with Implant Materials

FIG.3.

Comparison of Mechanical Properties of
Ti-13%Zr-13%Nb Alloy with Implant Materials

□=Ultimate Tensile Strength ▨=Yield Strength
▦=Compressive Strength

WROUGHT Ti-6Al-4V

316 SS (30%CW)

CAST Co-Cr-Mo

316 SS

Ti-13Zr-13Nb

C/PEEK COMPOSITE

C/PS COMPOSITE

CORTICAL BONE

STRENGTH, MPa

STRENGTH, ksi

MATERIAL

EP 0 437 079 B1

FIG. 4.

HARDNESS vs. AGING CONDITIONS FOR
Ti-13Zr-13Nb and Ti-18Zr-6Nb
ALLOYS COOLED AT TWO DIFFERENT RATES
FROM ABOVE THE BETA TRANSUS.